# EUROPEAN PATENT APPLICATION

(11) **EP 1 582 220 A1**
(43) Date of publication of application: **05.10.2005**
(21) Application number: 03778778.5
(22) Date of filing: 10.12.2003
(51) Int. Cl.: A61K 45/06, A61K 31/138, A61K 31/167, A61K 31/495, A61P 1/02, A61P 23/02, A61P 43/00

(54) **COMPOSITION FOR TOPICAL ANESTHESIA**

(30) Priority: 10.12.2002 JP 2002357823
(71) Applicant: Showa Yakuhin Kako Co., Ltd, Tokyo 104-0031 (JP)
(72) Inventor: HARAGUCHI, Mitsuhiro, SHOWA YAKUHIN KAKO CO., LTD., Tokyo 104-0031 (JP); KAWASAKI, Yoshihiko, SHOWA YAKUHIN KAKO CO., LTD., Tokyo 104-0031 (JP)
(74) Representative: Godemeyer, Thomas
(86) International application number: PCT/JP2003/015794
(87) International publication number: WO 2004/052399

(57) **Abstract**

A composition for local anesthesia which comprises a local anesthetic such as lidocaine hydrochloride as an active ingredient and an agent for sustaining anesthetic action selected from the group consisting of antihistamines such as diphenhydramine hydrochloride or hydroxyzine hydrochloride, and does not substantially contain catecholamines such as epinephrine, which has increased durability of anesthetic action without using catecholamines, and is useful as a safe composition for local anesthesia for performing short-time dental operations such as tooth extraction or oral surgery operations.

## Description

### Technical Field

The present invention relates to a composition for local anesthesia. More specifically, the present invention relates to a safe composition for local anesthesia which has durability of action suitable for minor dental operations such as tooth extraction.

### Background Art

For operations in the fields of oral surgery and dental treatment, in particular, for tooth extraction and the like in the field of dental treatment, anesthetics for local injection (agents for local anesthesia) containing lidocaine (2-diethylamino-N-(2,6-dimethyl-phenyl)acetamide) as an active ingredient have been used. For example, "Xylocaine Cartridge for Dental Use" (Fujisawa Pharmaceutical Co., Ltd.) has been clinically used. This agent for local anesthesia is a composition for topical administration which contains 20 mg of lidocaine hydrochloride and 0.0125 mg of epinephrine per 1 ml of a solution for injection. The agent is generally used in an amount of 0.3-1.8 ml to carry out infiltration anesthesia or block anesthesia (see, a package insert of the drug).

Agents for local anesthesia are generally formulated with a catecholamine such as epinephrine which has angiotonic effect on local capillary blood vessels to reduce blood flow. The effect of the catecholamine is to decrease bleeding in a field of operation by lowering blood flow, and to reduce transmigration (diffusion) of an anesthetic agent being an active ingredient into blood and maintain high concentration of the anesthetic agent in the local tissue to achieve a prolonged local anesthetic effect (Collins, V. J., Principles of Anesthesiology, 2nd Ed., Lea and Febiger, Philadelphia, 1976; as a review about agents for dental local anesthesia, see, Dental Outlook, special edition, "Medical practice of tooth extraction," 4. Dental local anesthetics, pp.84-94, 1979).

However, because epinephrine contained in topically administered anesthetics may possibly cause vasoconstriction in other tissues or in the whole body, it has been so far pointed out that local anesthetics containing epinephrine have possibilities of danger for administration to patients with hypertonia, anteriosclerosis, cardiac failure, hyperthyreosis, or diabetes or a patient who has experienced angiospasm. Therefore, the administration of the drug is a contraindication in principle (The term "contraindication in principle" means that an administration to the above patients is not allowed in principle, and when an administration is particularly required, the administration needs to be performed very carefully: Announcement in June, 2000, by Chief of safety measure Division of Pharmaceutical and Medical Safety Bureau of Ministry of Health and Welfare).

In dental lidocaine preparations which are clinically used, epinephrine is mixed at 1/80,000 (g/ml, 0.0125 mg per ml). For the purpose of decreasing side effects of epinephrine, anesthetics for dental use containing about 1/200,000 (g/ml) of epinephrine (0.005 mg as a free base per ml) are proposed as compositions for local anesthesia having durability suitable for short-time dental operations such as tooth extraction (WO 97/07794). By using said anesthetics, necessary and sufficient durability of anesthetic action for minor dental operations and the like can be achieved, however, the possibility of side effects of epinephrine cannot be completely eliminated. Therefore, other means for sustaining the action of local anesthetics are desired to be provided.

As for agents for sustaining the action of local anesthetics, it is known that substances selected from the group consisting of acidic mucopolysaccharides such as sodium chondroitin sulfate and cellulose derivatives such as hydroxypropylmethylcellulose have the action of sustaining anesthetic action of local anesthetics such as lidocaine hydrochloride (WO 02/055107). However, almost no substance useful as the agent for sustaining the action of local anesthetics has been known so far other than the aforementioned substances.

It is known that diphenhydramine hydrochloride, which is an antihistamine, has a local anesthetic action. However, it has not been reported so far that this substance, per se, maintains the action of local anesthetics such as lidocaine hydrochloride. Further, solutions for external use, aerosols, ointments and the like for therapeutic treatment of dermatosis containing diphenhydramine hydrochloride and lidocaine hydrochloride are known (Japanese Patent Publication (KOKOKU) No. 7-74152, Japanese Patent Unexamined Publication (KOKAI) No. 11-228398, Japanese Patent Publication No. 61-46451 and the like). However, all of these preparations are provided for a purpose of using the antihistaminic action per se of diphenhydramine hydrochloride, and the diphenhydramine hydrochloride is not added to these preparation for the purpose of sustaining the local anesthetic action of lidocaine hydrochloride.

### Disclosure of the Invention

An object of the present invention is to provide a composition for local anesthesia which has excellent durability of action. More specifically, the object of the present invention is to provide a safe composition for local anesthesia which has durability of action suitable for minor dental operations such as tooth extraction and oral surgery operations without using catecholamines.

The inventors of the present invention conducted intensive researches to achieve the foregoing objects, and as a result, they found that antihistamines such as diphenhydramine hydrochloride and hydroxyzine hydrochloride have a function to significantly maintain anesthetic action of local anesthetics such as lidocaine hydrochloride. The present invention was achieved on the basis of these findings.

The present invention thus provides a composition for local anesthesia which comprises a local anesthetic as an active ingredient and an agent for sustaining anesthetic action selected from the group consisting of antihistamines, and does not substantially contain catecholamines. The aforementioned composition for local anesthesia is preferably provided as a composition for local anesthesia used for oral surgery or dental treatment. According to the preferred embodiment of the present invention, there are provided the aforementioned composition for local anesthesia, wherein the local anesthetic is lidocaine hydrochloride; the aforementioned composition for local anesthesia wherein, the antihistamine is an antihistamine having a diphenylmethyl group (the phenyl groups may be substituted or unsubstituted) as a partial structure; and the aforementioned composition for local anesthesia, wherein the antihistamine is diphenhydramine hydrochloride or hydroxyzine hydrochloride.

From another aspect, the present invention provides an agent for sustaining anesthetic action of a local anesthetic selected from the group consisting of antihistamines. According to the preferred embodiments of the present invention, there are provided the aforementioned agent for sustaining action, wherein the local anesthetic is lidocaine hydrochloride; and the aforementioned agent for sustaining action, wherein the antihistamine is diphenhydramine hydrochloride or hydroxyzine hydrochloride.

From a further aspect, the present invention provides use of a substance selected from the group consisting of antihistamines for manufacture of the aforementioned composition for local anesthesia; and a method for sustaining anesthetic action of a local anesthetic comprising the step of topically administering a substance selected from the group consisting of antihistamines together with a local anesthetic.

### Brief Explanation of the Drawings

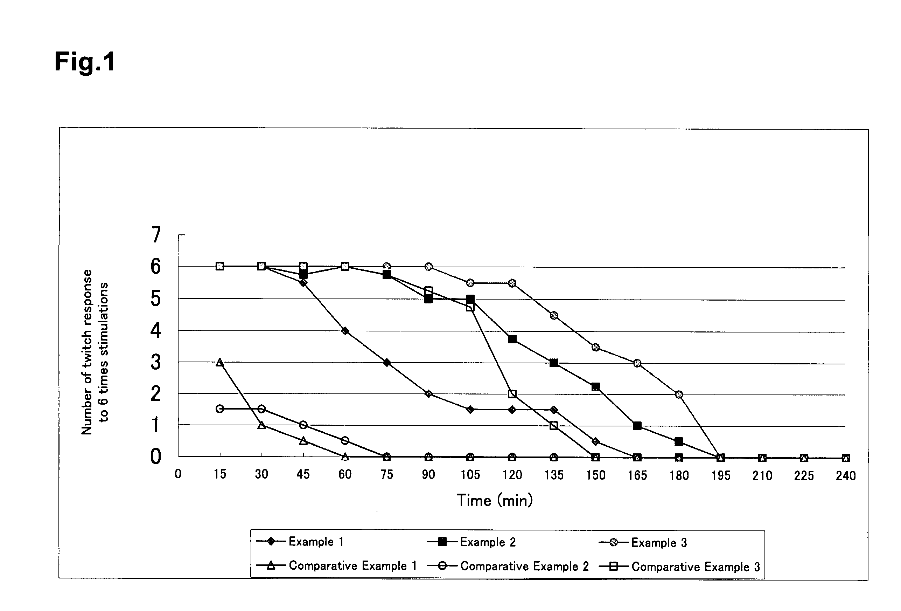

Fig. 1 shows sustaining effect of the composition for local anesthesia of the present invention. The results obtained by using an anterior portion of back skin of a guinea pig are shown.

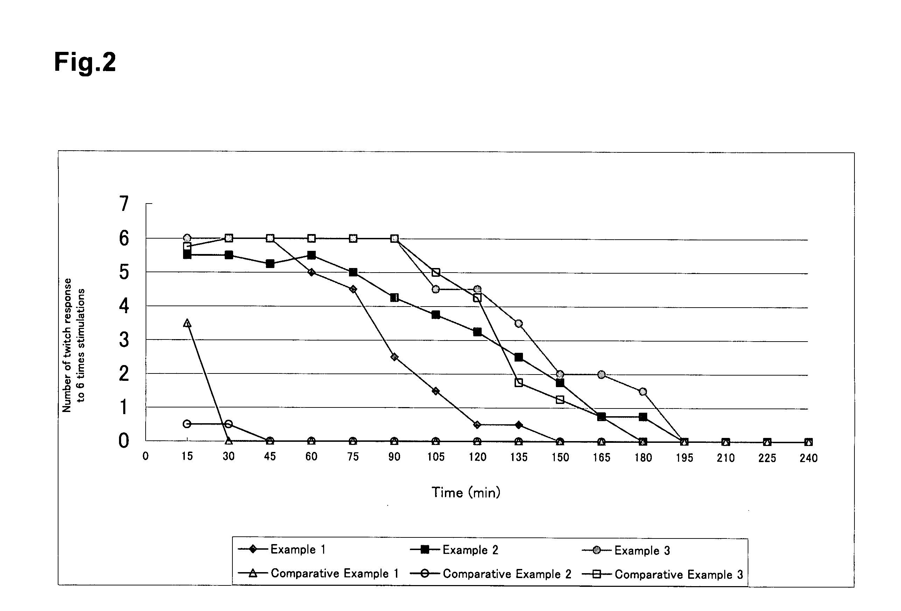

Fig. 2 shows sustaining effect of the composition for local anesthesia of the present invention. The results obtained by using a posterior portion of back skin of a guinea pig are shown.

### Best Mode for Carrying Out the Invention

Types of the local anesthetics which are comprised in the composition of the present invention are not particularly limited. Examples include cocaine analogues such as cocaine and tropacocaine; water-soluble esters of aminobenzoic acid such as procaine and tetracaine; esters of benzoic acid such as piperocaine and stovaine; esters of alkoxybenzoic acid such as cyclomethycaine and parethoxycaine; aminoketones such as dyclonine and falicaine; aminoethers such as pramoxine; benzofuranone derivatives such as amolanone; amidine or guanidine derivatives such as phenacaine; urethane derivatives such as diperodon; quinoline or isoquinoline derivatives such as dibucaine; amino acid anilides such as lidocaine; alkylesters of aminobenzoic acid such as ethyl aminobenzoate.

The classification of the aforementioned local anesthetics is described for convenience according to the classification described in Table 32 at pages from 202 to 205 in "Pharmacology" co-edited by Takagi and Ozawa, published by Nanzan-doh Co., Ltd. as the second issue in 1976. However, it should be understood that classifications other than the above are acceptable and the active ingredient of the composition of the present invention is not limited to the above examples. These local anesthetics are generally used in forms of physiologically acceptable salts. Examples of such salts include mineral acid salts such as hydrochloride and sulfate. Among them, a local anesthetic selected from the group consisting of lidocaine, procaine, tetracaine, dibucaine, and salts thereof may preferably be used. More preferably, a local anesthetic selected from the group consisting of lidocaine hydrochloride, procaine hydrochloride, tetracaine hydrochloride, and dibucaine hydrochloride may be used. Lidocaine hydrochloride may most preferably be used.

An antihistamine generally means an agent having affinity for a histamine receptor and antagonistically acting on the action of histamine by blocking or suppressing the action of histamine. Type of the antihistamine used in the composition of the present invention as an agent for sustaining action of local anesthetics is not particularly limited. As the antihistamine, for example, preferred examples include those having, as a partial structure, a diarylmethyl group (two of the aryl groups in the group may be the same or different, and may be, for example, substituted or unsubstituted phenyl groups, or substituted or unsubstituted heteroaryl groups containing a heteroatom as a ring-constituting atom such as pyridyl group), preferably a diphenylmethyl group (each of the two phenyl groups in the group may be substituted or unsubstituted). As a substituent on the phenyl groups, halogen atoms such as chlorine atom are preferred. It is preferred that one chlorine atom substitutes on one phenyl group of the two phenyl groups, and it is more preferred that the chlorine atom is present in the para-position. It is also preferred that two of the phenyl groups are unsubstituted.

More specifically, examples of the antihistamine include, for example, diphenhydramine, chlorphenoxamine, carbinoxamine, chlorpheniramine, tolopropamine, homochlorcyclizine, diphenylpyraline, clemastine, hydroxyzine, meclizine, diphenidol and the like. Among them, diphenhydramine and hydroxyzine are preferred. Two or more kinds of antihistamines can also be used in combination. These antihistamines can be used as pharmacologically acceptable salts. Types of the pharmacologically acceptable salts can be suitably chosen by those skilled in the art, and specific examples include, for example, diphenhydramine hydrochloride, diphenhydramine salicylate, hydroxyzine hydrochloride and the like.

An amount of the agent for sustaining anesthetic action selected from the group consisting of antihistamines can be suitably selected depending on the type of the agent for sustaining anesthetic action, the type of the local anesthetic, the desired duration, anesthetic depth of local anesthesia, and the like, and generally selected to be in the range of approximately 0.1 g to 10 g based on 1 g of local anesthetic. Anesthetic duration of the composition for local anesthesia can be objectively and precisely determined by the methods described in, for example, Journal of Japanese Dental Society of Anesthesiology, 16, pp. 10-22, 1988; and Journal of Japanese Dental Society of Anesthesiology, 27, pp. 158-164, 1999, or the method specifically described in Examples of the present specification.

The composition for local anesthesia of the present invention can be provided as a composition for injection in a form of an aqueous solution in which the aforementioned components and optional pharmaceutical additives, which are available for those skilled in the art as additives to be formulated in compositions for topical injections, are dissolved in distilled water for injection. The composition for local anesthesia of the present invention can also be prepared as a pharmaceutical preparation in a dried form such as a lyophilized preparation, and dissolved when used. Generally, the composition is provided for clinical use after being filled in ampoules, vials, cartridges or the like under sterile condition. As the pharmaceutical additives, for example, isotonicities to adjust osmotic pressure ratio to about 0.8-1.3, preferably about 1.0, e.g., sodium chloride; pH modifiers to adjust pH to a range of about 3.0-7.5, preferably 3.3-7.0, e.g., hydrochloric acid or sodium hydroxide; antiseptics, e.g., methyl p-hydroxybenzoate, and the like may be used.

The composition for local anesthesia of the present invention can be suitably used for minor operations in oral surgery and dental treatment, preferably for operations which can be completed in several to ten minutes such as tooth extraction in dental treatment. However, applicable operations are not limited to the uses in the oral surgery and dental treatment, and the composition can be used for surgical local anesthesia such as for skin incision. The agent for sustaining action of a local anesthetic contained in the composition of the present invention prolongs duration of the action of a local anesthetic, and has an effect of increasing anesthetic depth as well. Therefore, the composition for local anesthesia of the present invention provides increased depth and durability of local anesthesia without using catecholamines such as epinephrine, and has a feature that the composition can be used as a safe local anesthetic even to patients with hypertonia, anteriosclerosis, cardiac failure, hyperthyreosis, or diabetes or a patient who has experienced angiospasm.

The composition of the present invention can be prepared by a method well known to those skilled in the art. Specific examples of the method for producing the composition of the present invention are detailed in the following examples. However, methods for preparing the composition of the present invention are not limited to those described in the examples, and appropriate alterations and modifications can be added to these methods.

### Examples

The present invention will be explained more specifically by referring to the following examples. However, the scope of the present invention is not limited to these examples.

### Example 1

Diphenhydramine hydrochloride (1 g) and lidocaine hydrochloride (2 g) were dissolved in distilled water for injection (80 ml). After being adjusted to pH 6 by adding a sufficient quantity of sodium hydroxide, the solution was added with distilled water for injection up to the total volume of 100 ml to prepare a composition for local anesthesia.

### Example 2

Diphenhydramine hydrochloride (2 g) and lidocaine hydrochloride (2 g) were dissolved in distilled water for injection (80 ml). After being adjusted to pH 6 by adding a sufficient quantity of sodium hydroxide, the solution was added with distilled water for injection up to the total volume of 100 ml to prepare a composition for local anesthesia.

### Example 3

Hydroxyzine hydrochloride (2.5 g) and lidocaine hydrochloride (2 g) were dissolved in distilled water for injection (80 ml). After being adjusted to pH 6 by adding a sufficient quantity of hydrochloric acid, the solution was added with distilled water for injection up to the total volume of 100 ml to prepare a composition for local anesthesia.

### Comparative Example 1

Lidocaine hydrochloride (2 g) was dissolved in distilled water for injection (80 ml). After being adjusted to pH 6 by adding a sufficient quantity of sodium hydroxide, the solution was added with distilled water for injection up to the total volume of 100 ml to prepare a composition for local anesthesia.

### Comparative Example 2

Diphenhydramine hydrochloride (3 g) was dissolved in distilled water for injection (80 ml). After being adjusted to pH 6 by adding a sufficient quantity of sodium hydroxide, the solution was added with distilled water for injection up to the total volume of 100 ml to prepare a composition for local anesthesia.

### Comparative Example 3

For comparison, a commercial local anesthetic containing lidocaine hydrochloride (2 g), epinephrine hydrogen tartrate (2.5 mg), and sodium pyrosulfite (60 mg) in 100 ml (pH 4) (ORA Inj. Cartridge, produced by Showa Yakuhin Kako Co., Ltd.) was used.

### Test method

Four to five-week old Hartley male guinea pigs having a body weight of 300 to 400 g were used. Hair in the back was shaved one day before the test so that a test substance was administrable at two positions of the anterior portion and posterior portion. A drug solution (0.1 ml) was intracutaneously injected at the positions of which hair was shaved (two positions of the anterior portion and posterior portion), and the positions around the intracutaneously produced papules were marked with a felt-tip pen. Outside of the papules was stimulated with a needle (injection needle: 27G), and occurrence of a constrictive reaction was confirmed. Then, inside of the papules was stimulated at six positions at intervals of 3 to 5 seconds, and the number of times which gave no constrictive reaction was counted. This stimulation operation was performed at intervals of 15 minutes for 180 minutes after the administration to determine durability of the local anesthetic effect. The results are shown in Table 1. From the results, it is clearly understood that the local anesthetic effect of the compositions for local anesthesia of the present invention was significantly prolonged.

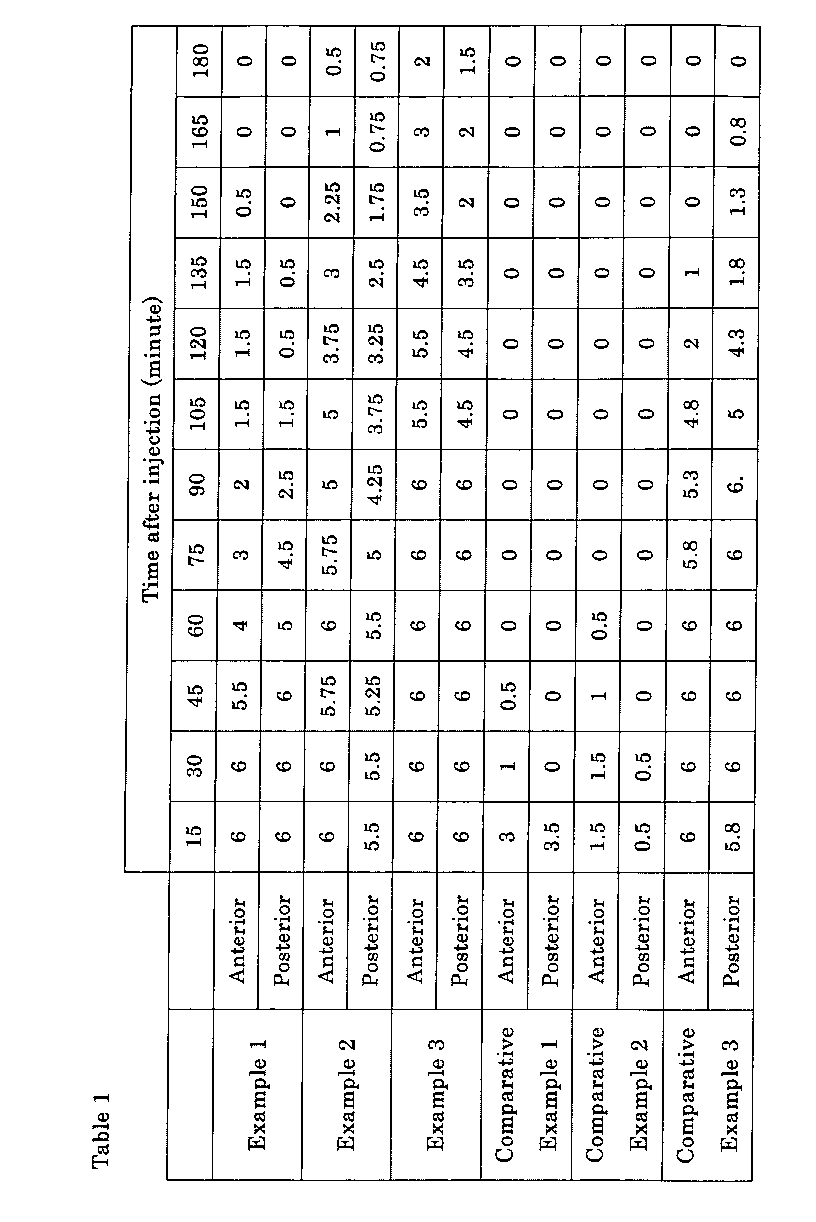

### Industrial Applicability

The composition for local anesthesia of the present invention provides increased anesthetic depth and durability of local anesthesia without using catecholamines such as epinephrine, and is useful as a safe composition for local anesthesia used for short-time dental operations such as tooth extraction and oral surgery operations.

## Claims

1. A composition for local anesthesia which comprises a local anesthetic as an active ingredient and an agent for sustaining anesthetic action selected from the group consisting of antihistamines, and does not substantially contain a catecholamine.

2. The composition for local anesthesia according to claim 1, which is used for oral surgery or dental treatment.

3. The composition for local anesthesia according to claim 1 or 2, wherein the local anesthetic is lidocaine hydrochloride

4. The composition according to any one of claims 1 to 3, wherein the antihistamine is an antihistamine having a diphenylmethyl group (the phenyl groups may be substituted or unsubstituted) as a partial structure.

5. The composition for local anesthesia according to claim 4, wherein the antihistamine is diphenhydramine hydrochloride or hydroxyzine hydrochloride.

6. An agent for sustaining action of a local anesthetic selected from the group consisting of antihistamines.

7. The agent for sustaining action according to claim 6, wherein the local anesthetic is lidocaine hydrochloride.
